# EUROPÄISCHE PATENTSCHRIFT

(11) **EP 1 370 663 B1**
(45) Veröffentlichungstag und Bekanntmachung des Hinweises auf die Patenterteilung: **07.03.2007**
(21) Anmeldenummer: 02712878.4
(22) Anmeldetag: 31.01.2002
(51) Int. Cl.: C12N 15/55, C12N 9/18, C12N 1/21, C12P 13/00, C12R 1/01, C12R 1/41

(54) **MIKROBIOLOGISCHES VERFAHREN ZUR HERSTELLUNG VON L-CARNITIN**
MICROBIOLOGICAL METHOD FOR PRODUCING L-CARNITINE
PROCEDE MICROBIOLOGIQUE POUR LA PRODUCTION DE L-CARNITINE

(30) Priorität: 31.01.2001 EP 01102214
(43) Veröffentlichungstag der Anmeldung: 17.12.2003
(73) Patentinhaber: Lonza AG, 4052 Basel (CH)
(72) Erfinder: BORNSCHEUER, Uwe, 17489 Greifswald (DE); MUSIDLOWSKA, Anna, 17489 Greifswald (DE); WERLEN, Josef, CH-3916 Ferden (CH); ZIMMERMANN, Thomas, CH-3904 Naters (CH); TSCHERRY, Benno, (CH)
(86) Internationale Anmeldenummer: PCT/EP2002/001009
(87) Internationale Veröffentlichungsnummer: WO 2002/061094

(56) Entgegenhaltungen:
- EP-A- 0 158 194
- EP-A- 0 360 222
- EP-A- 0 543 344
- WO-A-95/10613
- NAIDU G S N ET AL: "Microbial and enzymatic production of L-carnitine." BIOPROCESS ENGINEERING, Bd. 23, Nr. 6, Dezember 2000 (2000-12), Seiten 627-635, XP008015119 ISSN: 0178-515X

## Beschreibung

Die vorliegende Erfindung betrifft ein Verfahren zur Herstellung von L-Carnitin aus Betain-estern. Die Erfindung betrifft ferner Hydrolasen, die Betain-ester zu den entsprechenden Betainen umsetzen.

L-Carnitin ist eine natürliche, vitaminähnliche Substanz, die den Transport von Fettsäuren durch die mitochondriale Membran vermittelt und somit den Abbau von Fettsäuren ermöglicht. L-Carnitin findet Anwendung als Nahrungsergänzungsmittel. Die Verabreichung von L-Carnitin kann eine unzureichende körpereigene L-Carnitin-Produktion, z. B. bei hoher sportlicher Belastung, kompensieren. Für Kleinkinder ist die Ergänzung der Nahrung mit L-Carnitin essentiell.

Es sind mehrere biotechnologische Verfahren zur Herstellung von L-Carnitin bekannt.

Die EP-A-0 158 194 beschreibt ein Verfahren zur Herstellung von L-Carnitin ausgehend von 4-Butyrobetain mittels Mikroorganismen, die befähigt sind, aus 4-Butyrobetain und/oder Crotonobetain L-Carnitin zu produzieren ohne letzteres zu katabolisieren.

Die WO-A-95/10613 beschreibt ein Verfahren zur Herstellung von L-Carnitin ausgehend von 4-Butyrobetain und/oder Crotonobetain mittels Mikroorganismen, die mit einem DNA-Fragment transformiert sind, das eines oder mehrere der im Butyrobetain/Crotonobetain-Stoffwechsel für die Enzyme der L-Carnitin-Biosynthese codierenden Gene *bco*C, *bco*A/B und *bco*D umfaßt.

Diese beiden Verfahren haben den Nachteil, dass die eingesetzten Edukte, 4-Butyrobetain und Crotonobetain, schwer zugänglich sind und daher einen Hauptkostenfaktor bei der L-Carnitin-Synthese darstellen. So beschreibt die EP-B-0 360 222 ein chemisches Verfahren zur Herstellung von 4-Butyrobetain ausgehend von 4-Butyrolacton, bei dem 4-Butyrolacton zunächst mittels HCl zu 4-Chlorbutyrobetain und dann weiter mit Ethanol und Trimethylamin zu 4-Butyrobetain-ethylester umgesetzt wird. Letzterer wird dann mittels verdünnter NaOH zu 4-Butyrobetain hydrolysiert und das 4-Butyrobetain mittels Elektrodialyse von nicht umgesetztem 4-Butyrobetain-ethylester abgetrennt.

Aufgabe der Erfindung ist es daher, ein Verfahren zur Herstellung von L-Carnitin bereit zu stellen, bei dem kein Betain als Ausgangsmaterial benötigt wird.

Die obige Aufgabe wurde mit einem biotechnologischen Verfahren gelöst, bei dem das gewünschte L-Carnitin ausgehend von Betain-estern hergestellt wird. Es wurden nämlich überraschend Hydrolasen gefunden, die in der Lage sind, Betain-ester als Substrate zu verwerten und zu den entsprechenden Betainen zu hydrolysieren, die dann weiter zu L-Carnitin umgesetzt werden können.

Gegenstand der vorliegenden Erfindung ist daher ein biotechnologisches Verfahren zur Herstellung von L-Carnitin, worin ein Betain-ester der allgemeinen Formel 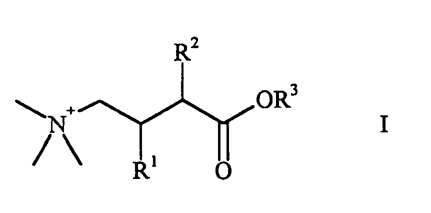 worin R¹ und R² entweder Wasserstoff bedeuten oder zusammen eine Bindung bedeuten und R³ gegebenenfalls substituiertes C₁₋₁₀-Alkyl, gegebenenfalls substituiertes Aryl oder gegebenenfalls substituiertes Arylalkyl bedeutet, mittels einer Hydrolase, die befähigt ist, einen Betain-ester der allgemeinen Formel I als Substrat zum entsprechenden Betain umzusetzen, oder mittels eines eine solche Hydrolase enthaltenden Mikroorganismus zum Betain der allgemeinen Formel 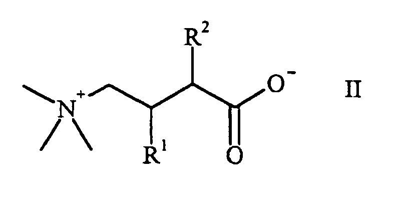 worin R¹ und R² die oben genannte Bedeutung haben, umgesetzt wird und das gebildete Betain der allgemeinen Formel II mittels eines Mikroorganismus, der befähigt ist, dieses Betain zu L-Carnitin umzusetzen, zu L-Carnitin umgesetzt wird.

Unter C₁₋₁₀-Alkyl sind hier und im folgenden sowohl lineare als auch verzweigte Alkylgruppen zu verstehen. Beispiele für C₁₋₁₀-Alkyl sind Methyl, Ethyl, Propyl, Isopropyl, Butyl, Isobutyl, *tert*-Butyl, Pentyl und seine Isomere, Hexyl und seine Isomere, Heptyl und seine Isomere, Octyl und seine Isomere, Nonyl und seine Isomere sowie Decyl und seine Isomere. Bevorzugt ist C₁₋₆-Alkyl, ganz besonders bevorzugt C₁₋₄-Alkyl. Die Alkylreste können unsubstituiert oder beispielsweise mit ein oder mehreren Hydroxylgruppen und/oder Halogenatomen substituiert sein. Unter Halogen werden hier und im folgenden Fluor, Chlor, Brom und Iod verstanden. Beispiele für Betain-ester der allgemeinen Formel I, worin R³ unsubstituiertes C₁₋₁₀-Alkyl ist, sind 4-Butyrobetain-methylester, 4-Butyrobetain-ethylester, 4-Butyrobetain-propylester, 4-Butyrobetain-isopropylester, 4-Butyrobetain-butylester, 4-Butyrobetain-*tert*-butylester und 4-Butyrobetain-isobutylester, Crotonobetain-methylester, Crotonobetain-ethylester, Crotonobetain-propylester, Crotonobetain-isopropylester, Crotonobetain-butylester, Crotonobetain-*tert*-butylester und Crotonobetain-isobutylester. Beispiele für Betain-ester der allgemeinen Formel I, worin R³ substituiertes C₁₋₁₀-Alkyl ist, sind 4-Butyrobetain-2-hydroxyethylester, 4-Butyrobetain-3-hydroxypropylester, 4-Butyrobetain-2,3-dihydroxypropylester, 4-Butyrobetain-2-chlorethylester, Crotonobetain-2-hydroxyethylester, Crotonobetain-3-hydroxypropylester, Crotonobetain-2,3-dihydroxypropylester und Crotonobetain-2-chlorethylester.

Unter Aryl sind hier und im folgenden insbesondere mono- oder bicyclische aromatische Reste zu verstehen, beispielweise Phenyl, 1-Naphthyl oder 2-Naphthyl, die sowohl unsubstituiert als auch substituiert sein können, beispielsweise mit ein oder mehreren Nitrogruppen und/oder Halogenatomen. Beispiele für Betain-ester der allgemeinen Formel I,
worin R³ unsubstituiertes oder substituiertes Aryl ist, sind 4-Butyrobetain-phenylester, 4-Butyrobetain-4-nitrophenylester, 4-Butyrobetain-4-chlorphenylester 4-Butyrobetain-1-naphthylester, Crotonobetain-phenylester, Crotonobetain-4-nitrophenylester, Crotonobetain-4-chlorphenylester und Crotonobetain-1-naphthylester.

Unter Arylalkyl sind hier und im folgenden entsprechend mit Aryl substituierte C₁₋₁₀-Alkylreste, insbesondere C₁₋₆-Alkylreste und bevorzugt C₁₋₄-Alkylreste zu verstehen,
worin Aryl die oben genannte Bedeutung hat und unsubstituiert oder wie oben substituiert sein kann. Bevorzugt ist Arylalkyl Benzyl, welches am Phenylring substituiert oder unsubstituiert ist, beispielsweise mit ein oder mehreren Nitrogruppen und/oder Halogenatomen. Beispiele für Betain-ester der allgemeinen Formel I, worin R³ Arylalkyl ist, sind 4-Butyrobetain-benzylester, 4-Butyrobetain-4-nitrobenzylester, 4-Butyrobetain-4-chlorbenzylester, Crotonobetain-benzylester, Crotonobetain-4-nitrobenzylester und Crotonobetain-4-chlorbenzylester.

Bevorzugt eingesetzte Betain-ester, die von der allgemeinen Formel I umfasst werden, sind 4-Butyrobetain-methylester, Crotonobetain-methylester, 4-Butyrobetain-ethylester, Crotonobetain-ethylester, 4-Butyrobetain-propylester, Crotonobetain-propylester, 4-Butyrobetain-2,3-dihydroxy-propylester, Crotonobetain-2,3-dihydroxy-propylester, 4-Butyrobetain-phenylester, Crotonobetain-phenylester, 4-Butyrobetain-benzylester und Crotonobetain-benzylester. Besonders bevorzugt sind 4-Butyrobetain-methylester, Crotonobetain-methylester, 4-Butyrobetain-ethylester, Crotonobetain-ethylester, 4-Butyrobetain-2,3-dihydroxy-propylester und Crotonobetain-2,3-dihydroxy-propylester. Ganz besonders bevorzugt sind 4-Butyrobetain-methylester und Crotonobetain-methylester.

Hydrolasen, die befähigt sind, Betain-ester, die unter die allgemeine Formel I fallen, insbesondere 4-Butyrobetain-methylester und/oder Crotonobetain-methylester, als Substrat zu den entsprechenden Betainen umzusetzen, wurden überraschend in den Mikroorganismen der Gattung *Agrobacterium*/*Rhizobium* gefunden, die bereits in der EP-A-0 158 194 und der WO-A-95/10613 beschrieben sind. Die neuen, zur Umsetzung von Betain-estern befähigten Hydrolasen sind noch nicht beschrieben und daher ebenfalls Gegenstand der Erfindung.

Die erfindungsgemäßen, beispielsweise aus Mikroorganismen der Gattung *Agrobacterium*/*Rhizobium* erhältlichen Hydrolasen sind befähigt, 4-Butyrobetain-methylester als Substrat zu 4-Butyrobetain umzusetzen. Sie sind durch einen isoelektrischen Punkt (pI) von 4,15 ± 0,30 sowie ein Molekulargewicht von 22,0 ± 2,0 kDa, bestimmt mittels SDS-PAGE, gekennzeichnet.

Diese Hydrolasen liegen in den Mikroorganismen der Gattung *Agrobacterium*/*Rhizobium* zusammen mit einer zweiten Hydrolase mit einem Molekulargewicht von 33 kDa vor, die jedoch nicht zur Umsetzung von Betainestern in der Lage ist. Sie läßt sich aus diesen Mikroorganismen nach fachmännisch bekannten Methoden isolieren und von der 33 kDA Hydrolase abtrennen.

Die erfindungsgemäßen Hydrolasen sind häufig in der Lage, neben dem 4-Butyrobetain-methylester noch andere unter die allgemeinen Formel I fallende Betain-ester, beispielsweise den Crotonobetain-methylester, als Substrat zum entsprechenden Betain umzusetzen.

Die erfindungsgemässen Hydrolasen sind bevorzugt aus Mikroorganismen der Gattung *Agrobacterium*/*Rhizobium,* insbesondere den Stämmen *Agrobacterium*/*Rhizobium* HK4 (DSM 2938), HK13 (DSM 2903), HK1331b (DSM 3225) und HK1349 (DSM 3944), erhältlich. Die Stämme HK4 (DSM 2938), HK13 (DSM 2903) und HK1331b (DSM 3225) werden in der EP-A-0 158 194 beschrieben. Der Stamm HK1349 (DSM 3944) wird in der EP-B-0 543 344 beschrieben. Der Stamm HK4 (DSM 2938) stellt den Wildtyp dar, die anderen Stämme sind im Gen *bco*E mutiert und L-Carnitin Dehydrogenase negativ. Die Stämme HK4 (DSM 2938), HK13 (DSM 2903), HK1331b (DSM 3225) und HK1349 (DSM 3944) weisen sowohl hohe Ähnlichkeit mit typischen Vertretern der Gattung *Agrobacterium* als auch mit typischen Vertretern der Gattung *Rhizobium* auf, konnten jedoch keiner dieser Gattungen eindeutig zugeordnet werden. Daher werden unter Mikroorganismen der Gattung *Agrobacterium*/*Rhizobium* sowohl die Mikroorganismen der Gattung *Agrobacterium* als auch die Mikroorganismen der Gattung *Rhizobium* sowie die Mikroorganismen der Gattung der Stämme HK4 (DSM 2938), HK13 (DSM 2903), HK1331b (DSM 3225) und HK1349 (DSM 3944) verstanden.

Der Stamm HK4 (DSM 2903) wurde am 03. April 1984, der Stamm HK13 (DSM 2903) am 23. Februar 1984, der Stamm HK1331b (DSM 3225) am 08. Februar 1985 und der Stamm HK1349 (DSM 3944) am 01. November 1991 bei Deutschen Sammlung für Mikroorganismen und Zellkulturen GmbH, Mascheroder Weg 1b, D-38124 Braunschweig, Deutschland gemäss Budapester Vertrag hinterlegt. Die Aufbewahrungsdauer des Mikroorganismus HK1349 (DSM 3944) ist nach Verlängerungsantrag bis zum 01. November 2031 garantiert.

Ferner wurde überraschend gefunden, daß auch bereits bekannte Hydrolasen, beispielsweise bestimmte Lipasen, Esterasen und Proteasen, befähigt sind, Betain-ester, die unter die allgemeine Formel I fallen, insbesondere 4-Butyrobetain-methylester und/oder Crotonobetain-methylester, als Substrat zu den entsprechenden Betainen umzusetzen. Diese Hydrolasen können ebenfalls in dem erfindungsgemäßen Verfahren eingesetzt werden.

Beispiele für bekannte Hydrolasen, die 4-Butyrobetain-methylester zu 4-butyrobetain umsetzen, sind Esterasen, Lipasen und Proteasen, insbesondere Pferdeleber-Esterase (erhältlich z. B. von Roche diagnostics oder Fluka), Schweineleber-Esterase (erhältlich z. B. von Roche diagnostics oder Fluka), Esterase Chirazym L2 (erhältlich z. B. von Roche diagnostics), Lipase aus *Bacillus thermocatenulatus* (erhältlich z. B. von Roche diagnostics), Lipase B von *Candida antartica* (erhältlich z. B. von Roche diagnostics), die Protease Subtilisin A60 (erhältlich z. B. von NovoNordisk), die Esterase PFE II aus *Pseudomonas fluorescens* (DSM 50106, Khalameyzer et al., *Appl. Environm. Microbiol.* **1999**, *65,* 477-482), die Esterase PFE I aus *Pseudomonas fluorescens* (Bornscheuer *et al., J. Biotechnol.* **1998**, *60,* 105-111) und die Esterase SDE aus *Streptomyces diastatochromogenes* (erhältlich z. B. von Jülich Fine Chemicals, Bornscheuer *et al., Biotechnol. Letters* **1999,** *21,* 101-104, Tesch *et al., J. Bacteriol.* **1996**, *178,* 1858-1865).

Besonders bevorzugt eingesetzte Hydrolasen sind die erfindungsgemässen neuen Hydrolasen aus *Agrobacterium*/*Rhizobium,* die Lipase aus *Bacillus thermocatenulatus,* die Esterase PFE II aus *Pseudomonas fluorescens* (DSM 50106) sowie die Esterase SDE aus *Streptomyces diastatochromogenes.*

Die Umsetzung des durch die Hydrolase gebildeten Betains der allgemeinen Formel II erfolgt mittels Mikroorganismen, die befähigt sind, dieses Betain zu L-Carnitin umzusetzen. Solche Mikroorganismen sind bereits im Stand der Technik beschrieben.

Beispiele für geeignete Mikroorganismen sind die bereits genannten Mikroorganismen der Gattungen *Agrobacterium*/*Rhizobium,* insbesondere der Stämme HK13 (DSM 2903), HK1331b (DSM 3225), HK1349 (DSM 3944) und HK1349 enthaltend Plasmid pVK100 q (DSM 8726), und Mikroorganismen der Gattungen *Pseudomonas, Achromobacter* oder *Escherichia.* Vorteilhafte Mikroorganismen sind diejenigen, die das gebildete Carnitin nicht katabolisieren, insbesondere Camitin-Dehydrogenase negative Stämme.

Die Umsetzung von Betain-ester zu Betain kann nicht nur mit freier Hydrolase, sondern vorteilhaft auch mit Mikroorganismen erfolgen, die die oben beschriebenen Hydrolasen enthalten und exprimieren. Dabei kann es sich sowohl um natürlich vorkommende Mikroorganismen handeln, in welchen das für die Hydrolase codierende Gen natürlicher Bestandteil des Genoms ist, als auch um rekombinante Mikroorganismen, die mit einer für eine solche Hydrolase codierenden DNA transformiert sind.

Erfolgt die Umsetzung des Betain-esters mit einem eine Hydrolase enthaltenden Mikroorganismus, so ist es vorteilhaft, wenn dieser Mikroorganismus gleichzeitig in der Lage ist, das gebildete Betain zu L-Carnitin umzusetzen. Der Mikroorganismus, der die Hydrolase enthält, ist dann gleichzeitig der Mikroorganismus, der das gebildetet Betain zu L-Carnitin umsetzt. Das erfindungsgemäße Verfahren kann dann ausgehend vom gewünschten Betain-ester der Formel I mit einem einzigen Mikroorganismus erfolgen. Vorteilhafte Mikroorganismen sind wiederum diejenigen, die das gebildete Carnitin nicht katabolisieren, insbesondere Carnitin-Dehydrogenase negative Stämme.

Soll die Umsetzung des Betain-esters nur bis zum Betain führen, so ist der Mikroorganismus vorteilhaft nicht in der Lage, das gebildete Betain zu verwerten und zu Carnitin umzusetzen.

Beispiele für Mikroorganismen, die das für die Hydrolase codierende Gen natürlich in ihrem Genom tragen, sind Mikroorganismen der Gattungen *Agrobacterium7Rhizobium, Bacillus, Pseudomonas* oder *Streptomyces.* Bevorzugte Mikroorganismen, dieser Art sind die Mikroorganismen der Gattung *Agrobacterium*/*Rhizobium,* insbesondere der Stämme HK13 (DSM 2903); HK1331b (DSM 3225), HK1349 (DSM 3944) oder HK1349 enthaltend Plasmid pVK100q (DSM 8726) und Mikroorganismen der Spezies *Bacillus thermocatenulatus, Pseudomonas fluorescens* oder *Streptomyces diastatochromogenes.*

Zur Herstellung rekombinanter Mikroorganismen können die für die obigen erfindungsgemässen und bekannten Hydrolasen codierenden Gene nach Standardmethoden kloniert werden. Hierfür können die Gene gegebenenfalls zuvor auf übliche Weise isoliert werden, indem man beispielsweise die N-terminale Aminosäuresequenz der gewünschten Hydrolase bestimmt und mit Hilfe der von dieser N-terminalen Aminosäuresequenz abgeleiteten DNA-Sonden das Hydrolase-Gen aus einer Genbank des entsprechenden Mikroorganismus isoliert. Anschließend wird ein geeigneter Wirtsorganismus mit einer in der Regel rekombinanten, das isolierte Gen enthaltenden DNA, vorteilhaft einer linearen oder ringförmigen Vektor-DNA transformiert. Der so erhaltene rekombinante Mikroorganismus kann das Hydrolase-Gen entweder nach homologer Rekombination integriert im Genom oder als extrachromosomales Element tragen.

Bevorzugt für die Transformation sind selbstreplizierende Vektoren, beispielsweise Expressionsvektoren, die die für die Hydrolase codierende Sequenz unter der Kontrolle geeigneter Regulationssequenzen enthält, also beispielsweise einem Promotor, welcher dem Hydrolase-Gen vorgeschaltet ist, und einer Ribosomenbindungsstelle. Promotor und Ribosomenbindungsstelle sind so gewählt, dass sie im jeweiligen Wirt funktionsfähig sind, so daß das Gen exprimiert wird. Je nach Wahl des Promotors erfolgt die Expression eines solchen Gens entweder konstitutiv oder induziert. Üblicherweise tragen diese Vektoren auch ein selektionierbares Marker-Gen, mit dem sich die Anwesenheit des Vektors im Wirt nach erfolgter Transformation nachweisen läßt. In einer besonderen Ausführungsform kann der Expressionsvektor zusätzlich die Gene für den Butyrobetain/Crotonobetain-Stoffwechsel umfassen, die beispielsweise in der WO-A-95/10613 beschrieben sind. Die Transkription dieser Gene wird dann entweder vom selben Promotor kontrolliert, der auch die Transkription des Hydrolase-Gens kontrolliert, oder von einem anderen geeigneten Promotor.

Beispiele für geeignete Promotoren sind die P_{L}- und P_{R}-Promotoren des Phagen Lambda (Schauter *et al., Gene* **1987,** *52,* 279-283,), der P_{trc}-Promotor (Amann *et al., Gene* **1988,** 69, 301-315), die P_{Nm}- und P_{S1}-Promotoren (Labes *et al., Gene* **1990,** *89,* 37-46), der Pₜᵣₚ-Promotor (Amann *et al., Gene* **1983,** 25, 167-178), der P_{lac}-Promotor (Amann *et al., Gene* **1983,** *25,* 167-178) und der P_{tac}-Promotor, (Russel und Bennet, *Gene* **1982,** 20, 231-243) und der Rhamnose-Promotor (Volff *et al., Mol. Microbiol.* **1996,** *21(5),* 1037-1047).

Beispiele für *Escherichia coli* spezifische Expressionsvektoren sind pBR322 (Bolivar *et al., Gene,* **1977,** *2(2),* 95-113), pBLUESCRIPT-KS+^{®} und pBLUESCRIPT-SK+^{®} (Stratagene), pUC18 und pUC19 (Yannisch-Perron et al., *Gene* **1985,** 33, 103-119), pK18 und pK19 (Pridmore, *Gene* **1987,** 56, 309-312), pRK290X (Alvarez-Morales *et al., Nucleic Acids Research, 14,* 4207-4227) und pRA95 (erhältlich z. B. von Nycomed Pharma AS, Huidove, Dänemark) und Derivate dieser Vektoren, die denselben Promotor, dieselbe ribosomale Bindungsstelle und denselben Replikationsursprung besitzen.

Beispiele für wirtsklassenspezifische Expressionsvektoren, die für gram-negative Wirtsorganismen spezifische sind, sind sogenannte "broad host range" Vektoren, beispielsweise die Plasmide pRK290 (Ditta *et al., PNAS* **1980,** *77,* 7347-7351), pKT240 (Bagdasarian *et al., Gene* **1983,** *26,* 273-282), das pGSS33 (Sharpe *et al., Gene* **1984,** *29,* 93-102), pVK100 (Knauf und Nester, *Plasmid* **1982,** *8,* 45-54), pME285 (Haas und Itoh, *Gene* 1985, 36, 27-36) und Derivate dieser Vektoren, die denselben Promotor, dieselbe ribosomale Bindungsstelle und denselben Replikationsursprung besitzen.

Als Wirt eignet sich jeder Mikroorganismus, der mit einer für die gewünschte Hydrolase codierenden DNA transformiert werden kann und nach dieser Transformation befähigt ist, diese Hydrolase zu exprimieren und somit einen Betain-ester zum Betain zu hydrolysieren.

Bevorzugte Wirte sind Mikroorganismen, die befähigt sind, aus 4-Butyrobetain und/oder Crotonobetain L-Carnitin zu produzieren. Geeignete Mikroorganismen sind beispielsweise solche, die die Gene für den Butyrobetain/Crotonobetain-Stoffwechsel natürlich in ihrem Genom tragen. Darunter sind diejenigen Wirte besonders bevorzugt, die das gebildete Carnitin nicht katabolisieren. Dabei handelt es sich beispielsweise um Stämme, die Carnitin-Dehydrogenase-negativ sind, beispielsweise um Stämme, die kein oder kein intaktes *bco*E-Gen enthalten.

Beispiele für geeignete Wirte sind die Mikroorganismen der Gattungen *Agrobacterium*/*Rhizobium, Pseudomonas* (Lindtstedt *et al, J. Bacteriol,* **1970,** *101(3),* 1094-*5), Achromobacter* (Naidu *et al., J. Ind Microbiol. Biotechnolog.* **2001**, *26(5),* 309-315) oder *Escherichia* (Jung *et al., Adv. Biochem. Eng.*/*Biotechnol.* **1993**, *50,* 21-44). Besonders bevorzugte Wirte dieser Art sind die Mikroorganismen der Gattung *Agrobaeterium*/*Rhizohium,* insbesondere die Stämme HK13 (DSM 2903), HK1331b (DSM 3225), HK1349 (DSM 3944) oder HK1349 enthaltend Plasmid pVK100 q (DSM 8726).

Rekombinante Mikroorganismen, die mit einer DNA transformiert sind, die für eine erfindungsgemäße Hydrolase codiert, die 4-Butyrobetain-methylester als Substrat zu 4-Butyrobetain umsetzt, sind im Stand der Technik nicht bekannt.

Rekombinante Mikroorganismen, die mit einer DNA transformiert sind, die für eine Hydrolase codiert, die 4-Butyrobetain-methylester als Substrat zu 4-Butyrobetain umsetzt,
wobei die Mikroorganismen befähigt sind aus 4-Butyrobetain-methylester L-Carnitin zu produzieren ohne letzteres zu katabolisieren, sind im Stand der Technik ebenfalls nicht bekannt.

Bevorzugte erfindungsgemässe Mikroorganismen sind Mikroorganismen der Gattung *Agrobacterium*/*Rhizobium,* insbesondere die Stämme der Gattungen *Agrobacterium*/*Rhizobium* HK13 (DSM 2903), HK1331b (DSM 3225), HK1349 (DSM 3944) oder HK1349 enthaltend Plasmid pVK100 q (DSM 8726), und der Gattungen *Pseudomonas, Achromobacter* oder *Escherichia,* die mit einer DNA transformiert sind, die für eine Hydrolase codiert, die 4-Butyrobetain-methylester zu 4-Butyrobetain hydrolysiert. Insbesondere ist die codierte Hydrolase eine erfindungsgemäße Hydrolase.

Bevorzugte Mikroorganismen, die eine Hydrolase enthalten, die einen 4-Butyrobetain-methylester als Substrat zum 4-Butyrobetain umsetzen und die in dem erfindungsgemäßen Verfahren eingesetzt werden können, sind:
a) Mikroorganismen der Gattung *Agrobacterium*/*Rhizobium,* bevorzugt der Stämme HK13 (DSM 2903), HK1331b (DSM 3225), HK1349 (DSM 3944) oder HK1349 enthaltend Plasmid pVK100q (DSM 8726), und Mikroorganismen der Gattung *Bacillus, Pseudomonas* oder *Streptomyces,* insbesondere der Spezies *Bacillus thermocatenulatus, Pseudomonas fluorescens* oder *Streptomyces diastatochromogenes.*
b) Rekombinante Mikroorganismen der Gattung *Agrobacterium*/*Rhizobium,* bevorzugt der Stämme HK13 (DSM 2903), HK1331b (DSM 3225), HK1349 (DSM 3944) oder HK1349 enthaltend Plasmid pVK100q (DSM 8726), und Mikroorganismen der Gattung *Pseudomonas, Bacillus, Achromobacter* oder *Escherichia,* die mit einer DNA transformiert sind, die für eine erfindungsgemäße Hydrolase codiert, die 4-Butyrobetain-methylester als Substrat zu 4-Butyrobetain umsetzt, sowie
c) Rekombinante Mikroorganismen der Gattung *Agrobacterium*/*Rhizobium,* bevorzugt der Stämme HK13 (DSM 2903), HK1331b (DSM 3225), HK1349 (DSM 3944) oder HK1349 enthaltend Plasmid pVK100q (DSM 8726), und Mikroorganismen der Gattung *Bacillus, Pseudomonas* oder *Streptomyces,* insbesondere der Spezies *Bacillus thermocatenulatus, Pseudomonas fluorescens* oder *Streptomyces diastatochromogenes,* die mit einer DNA transformiert sind, die für eine bekannte Lipase, Esterase oder Protease codiert, die 4-Butyrobetain-methylester als Substrat zu 4-Butyrobetain umsetzt, insbesondere die Lipase aus *Bacillus thermocatenulatus,* die Esterase PFE II aus *Pseudomonas fluorescens* (DSM 50106) sowie die Esterase SDE aus *Streptomyces diastatochromogenes.*

Die Anzucht der Mikroorganismen kann in einem Medium erfolgen, das geeignete Induktoren zur Induktion der Enzyme des Butyrobetain/Crotonobetain-Stoffwechsels enthält. Gegebenefalls kann den Medien zur Induktion der Hydrolase ein Betain-ester der allgemeinen Formel I zugesetzt werden.

Beispielsweise können die Mikroorganismen, bei welchen der Wirt einer der *Agrobacterium*/*Rhizobium*-Stämme HK13 (DSM 2903), HK1331b (DSM 3225), HK1349 (DSM 3944) und HK1349 enthaltend Plasmid pVK100 q (DSM 8726) ist, in "Nutrient Yeast broth" Vorkulturmedium, welchem L-Carnitin als Induktor der Enzyme des 4-Butyrobetain/ L-Carnitinstoffwechsels zugesetzt wurde, vorkultiviert werden und dann in einem Minimalmedium, welches Betain, Glycerin und L-Glutamat als C-Quellen enthält, angezogen werden.

Bevorzugt wird das erfindungsgemässe Verfahren so durchgeführt, dass der Betain-ester der allgemeinen Formel I so zum Reaktionsgemisch zugeführt wird, dass die Konzentration dieses Betain-esters im Reaktionsgemisch unter 10% (w/v) bleibt.

Bevorzugt werden die beiden erfindungsgemässen Verfahren bei einem pH-Wert im Bereich von 6 bis 8 und einer Temperatur im Bereich von 15 bis 50°C, beispielsweise von 25 bis 35°C durchgeführt.

Die erfindungsgemässe Herstellung von L-Carnitin aus einem Betain-ester der allgemeinen Formel I kann entweder mit wachsenden Mikroorganismen oder mit ruhenden Mikroorganismen durchgeführt wird. Bevorzugt wird das erfindungsgemässe Verfahren so durchgeführt, dass die Herstellung von L-Carnitin in der spätlogarithmischen Wachstumsphase der Mikroorganismen oder mit ruhenden Mikroorganismen erfolgt.
Das erhaltene L-Carnitin wird nach Standardmethoden aufgearbeitet.

Die erfindungsgemässe Verwendung von Hydrolasen, die 4-Butyrobetain-methylester zu 4-Butyrobetain umsetzen, in einem Verfahren zur Herstellung von L-Carnitin, bezieht sich sowohl auf die Verwendung von erfindungsgemässen Hydrolasen als auch auf die Verwendung von bekannten Hydrolasen, die diese Eigenschaft haben. Bevorzugt ist die Verwendung von erfindungsgemässen Hydrolasen sowie von bekannten Lipasen, Esterasen und Proteasen, die 4-Butyrobetain-methylester zu 4-Butyrobetain umsetzen, beispielsweise der Lipase aus *Bacillus thermocatenulatus,* der Esterase PFE II aus *Pseudomonas fluorescens* (DSM 50106) sowie der Esterase SDE aus *Streptomyces diastatochromogenes.*

Die erfindungsgemässe Verwendung von Mikroorganismen, welche eine Hydrolase enthalten, die 4-Butyrobetain-methylester zu 4-Butyrobetain umsetzt, in einem Verfahren zur Herstellung von L-Carnitin, bezieht sich sowohl auf die Verwendung natürlich vorkommender Mikroorganismen als auch auf die Verwendung rekombinanter Mikroorganismen.

Abb. 1 stellt die Abhängigkeit der relativen spezifischen Hydrolase-Aktivität, bestimmt mittels Essigsäure-4-nitrophenylester-Assay, der Hydrolasen aus HK1349 (DSM 3944) vom pH-Wert in 50 mM Natriumphosphat-Puffer bei 25°C dar.

Abb. 2 stellt die relativen spezifischen Hydrolase-Aktivitäten, bestimmt mittels Essigsäure-4-nitrophenylester-Assay, der Hydrolasen aus HK1349 (DSM 3944) nach 16 stündiger Inkubation bei Temperaturen von 5 bis 70°C in 50 mM Natriumphosphat-Puffer bei pH 7,5 dar.

Abb 3 stellt die SDS-PAGE-Gele der aus HK1349 (DSM 3944) erhaltenen Hydrolasen mit den Molekulargewichten von 22 kDa (Fraktion B und C) und 33 kDa (Fraktion A) dar. Die Detektion erfolgte sowohl mit Aktivitätsfärbung als auch mit Coomassie-Blue plus.

Abb. 4 stellt die Bestimmung der pI von der 22 kDa Hydrolase (Fraktion C) und der 33 kDa Hydrolase (Fraktion A) mittels PhastSystems (Amersham Pharmacia) dar. Die Detektion erfolgte sowohl mit Aktivitätsfärbung als auch mit Coomassie-Blue.

### Medien

| **Spurenelementlösung** | |
|---|---|
| EDTA | 500 mg/L |
| FeSO4 × 7 H₂O | 200 mg/L |
| ZnSO4 × 7 H₂O | 10 mg /L |
| MgCl₂ × 4 H₂O | 3 mg/L |
| H₃BO₃ | 30 mg/L |
| CaCl₂ × 6 H₂O | 20 mg/L |
| CuCl₂ × 2 H₂O | 1 mg/L |
| NiCl₂ × 2 H₂O | 2 mg/L |
| Na₂MoO₄ × 2 H₂O | 3 mg/L |

Die Lösung wurde bei 121°C und 1 bar Überdruck während 30 Minuten autoklaviert.

| **Vitaminlösung** | |
|---|---|
| Pyridoxal-Hydrochlorid | 10 µg/L |
| Lactoflavin | 5 µg/L |
| Nikotinamid | 5 µg/L |
| Thiamin-Hydrochlorid | 5 µg/L |
| Biotin | 2 µg/L |
| D-Pantothensäure Calciumsalz | 5 µg/L |
| 4-Aminobenzoesäure | 5 µg/L |
| Folsäure | 2 µg/L |
| Cyanocobalamin | 5 µg/L |

Die Lösung wurde steril filtriert.

| **Lösung B** | |
|---|---|
| MgCl₂ × 6 H₂O | 16 g |
| CaCl₂ × 2 H₂O | 580 mg |
| FeCl₃ × 6 H₂O | 32 mg |

Die obigen Komponenten wurden in destilliertem Wasser (1 L) gelöst. Die Lösung wurde mit konzentrierter Salzsäure (2 bis 3 Tropfen) versetzt und dann bei 121°C und 1 bar Überdruck während 30 Minuten autoklaviert.

| **(A+N)-Lösung** | |
|---|---|
| (NH₄)₂SO₄ | 20 g |
| Na₂HPO₄ × 2 H₂O | 25 g |
| KH₂PO₄ | 10 g |
| NaCl | 30 g |

Die Komponenten wurden in destilliertem Wasser (1 L) gelöst. Die Lösung wurde bei 121°C und 1 bar Überdruck während 30 Minuten autoklaviert.

| **Minimalmedium** | |
|---|---|
| Spurenelementlösung | 0,5 mL |
| Lösung B | 12,5 mL |

Die Komponenten wurden mit 450 mL destilliertem Wasser aufgefüllt, gegebenfalls wurde eine oder mehrere C-Quellen zugefügt und der pH-Wert wurde mit NaOH auf 7,0 eingestellt. Die Lösung wurde bei 121°C und 1 bar Überdruck während 30 Minuten autoklaviert, auf 45°C abgekühlt und mit

| | |
|---|---|
| (A+N)-Lösung | 50,0 mL |
| Vitaminlösung | 0,5 mL |

versetzt.

| **Minimalmedium Agarplatte** | |
|---|---|
| Spurenelementlösung | 0,5 mL |
| Lösung B | 12,5 mL |

Die Komponenten wurden mit destilliertem Wasser (450 mL) aufgefüllt, Agar (7,5 g) wurde zugegeben, gegebenfalls wurde eine oder mehrere C-Quellen zugefügt und der pH-Wert wurde mit NaOH auf 7,0 eingestellt. Die Lösung wurde bei 121°C und 1 bar Überdruck während 30 Minuten autoklaviert, auf 45°C abgekühlt und mit

| | |
|---|---|
| (A+N)-Lösung (45°C) | 50,0 mL |
| Vitaminlösung | 0,5 mL |

versetzt. Die noch warme (45°C) Lösung wurde in Petri-Schalen (20 bis 25 mL Lösung/Petri Schale) gegossen.

### Analytik

Die Bestimmung von Butyrobetain-methylester (BBMe), Butyrobetain-ethylester (BBEt), Butyrobetain und L-Carnitin mittels HPLC erfolgte unter folgenden Bedingungen: HPLC: Waters LC-Module I plus; Säule: Hamilton pRP-X200 250 × 4,1 mm; Durchflussrate: 1,3 mL/min; Detektion: Leitfähigkeit. Butyrobetain und L-Carnitin wurden mit 5 mM HClO₄ in Acetonitril:Wasser = 1:24 (v/v) eluiert. Butyrobetain-methylester (BBMe) und Butyrobetain-ethylester (BBEt) wurden mit 5 mM HClO₄ in Acetonitril:Wasser = 1:4 (v/v) eluiert.

Die Bestimmung von Butyrobetain-methylester (BBNME) und Butyrobetain mittels Dünnschichtchromatographie erfolgte unter folgenden Bedingungen: Cellulose-Platten; Laufmittel: Butanol:Essigsäure:Wasser = 8:4:2 (v/v/v); Detektion: Ioddampf.

Die Bestimmung der Hydrolase-Aktivität erfolgte photometrisch mittels Essigsäue-4-nitrophenylester-Assay. Hierzu wurde die Proteinlösung (900 µL) mit einer Lösung von Essigsäure-4-nitrophenylester (10 mM in Dimethylsulfoxid; 100 µL) bei 25°C versetzt. Die Absorption des während der Hydrolyse gebildeten 4-Nitrophenols (e = 15 × 10³ × M⁻¹ cm⁻¹) wurde bei 410 nm gemessen. 1 Unit (U) entspricht der Menge 4-Nitrophenol (in µmol), die pro Minute freigesetzt wird. Die Bestimmung des Proteingehaltes der eingesetzten Proteinlösung erfolgte mit dem BCA (Bicin choninic acid) Protein Assay kit (Pierce) nach Anleitung des Herstellers. Die spezifische Hydrolase-Aktivität wird angegeben in U/mg Protein und entspricht der Menge 4-Nitrophenol (in µmol), die pro Minute und mg Protein freigesetzt wird.

Die Bestimmung des Molekulargewichtes der Hydrolase erfolgte mittels SDS-Polyacrylamid-Gelelektrophorese (SDS-PAGE) (Puffer: Tris (0,025 M), Glycine (0,192 M), SDS (0,1% (w/v)); pH 8,4; Sammelgel: 10 mA, Trenngel: 25 mA). Die Detektion erfolgte sowohl mit Coomassie-Blue als auch mit Aktivitätsfärbung. Die Aktivitätsfärbung wurde wie folgt ausgeführt: Das Gel wurde in Renaturierungslösung (Triton X (0,5% (w/v)) in Tris × HCl-Puffer (0,1 M; pH 7,5) 30 min bei 25°C inkubiert. Dann wurde Essigsäure-1-naphthylester-Lösung (10 mL) und Fast Red-Lösung (10 mL) hinzugegeben. Die Hydrolasen färbten sich nach ca. 30 min rot-braun. Die Essigsäure-1-naphthylester Lösung wurde wie folgt hergestellt: Essigsäure-1-naphthylester (4 mg) wurde in Aceton (1,0 mL) gelöst und mit Tris × HCl-Puffer (0,1 M; pH 7,5) auf 10 mL aufgefüllt. Die Fast Red-Lösung wurde wie folgt hergestellt: Fest Red (10 mg) wurde in Tris × HCl-Puffer (0,1 M; pH 7,5; 10 mL) gelöst.

### Beispiel 1

### Wachstumsverhalten des Stammes HK4 (DSM 2938) in Medien enthaltend Butyrobetain-methylester (BBMe) und Butyrobetain-ethylester (BBEt) als jeweils einzige C-Quelle

Der Stamm HK4 (DSM 2938) wurde auf Minimalmedium Agarplatten unter Zusatz von 0,4% (w/v) Betain als einziger C-Quelle nach Einzelkolonie-Ausstrich bei 30°C für 48 bis 72 h inkubiert. Zur Anzucht wurden Einzelkolonien des Stammes von der Agarplatte in das Minimalmedium enthaltend Butyrobetain-methylester (BBMe) und Butyrobetain-ethylester (BBEt) als jeweils einziger C-Quelle überimpft und bei 30°C und 140 upm für 48 bis 96 h inkubiert.
Es wurde ein Kontrollversuch durchgeführt, bei dem die Anzucht des Stammes HK4 (DSM 2938) unter den gleichen Bedingungen, jedoch unter Zusatz von Butyrobetain anstelle eines Butyrobetain-esters als einziger C-Quelle, erfolgte.

Die Ergebnisse sind in Tabelle 1 zusammengefasst.

**Tabelle 1:**

| | **1,0 % (w/v) BBMe** | **0,2 % (w/v) BBEt** | **1,0 % (w/v) BBEt** | **1,0 % (w/v) Butyrobetain** |
|---|---|---|---|---|
| **Stamm** | **Wachstum [OD₆₅₀]** | | | |
| HK4 | 0,76 | | | |
| HK4 | | 0,23 | | |
| HK4 | | | 0,76 | |
| HK4 | | | | 7,0 |

Der Stamm HK4 (DSM 2938) wuchs in dem Minimalmedium enthaltend Butyrobetain-methylester (BBMe) und Butyrobetain-ethylester (BBEt) als jeweils einziger C-Quelle. Das Wachstum des Stammes HK4 (DSM 2938) auf Butyrobetain-ester als einziger C-Quelle war jedoch deutlich geringer verglichen mit dem Wachstum auf Butyrobetain als einziger C-Quelle.

### Beispiel 2

### Umsetzung von Butyrobetain-methylester (BBMe) und Butyrobetain-ethylester (BBEt) mittels der Stämme HK13 (DSM 2903) und HK1349 (DSM 3944) zu Butyrobetain und L-Carnitin, Dauer: 74 h

Die Stämme HK13 (DSM 2903) und HK1349 (DSM 3944) wurden jeweils in "Nutrient Yeast broth" Vorkulturmedium (10 mL), welchem L-Carnitin (0,05% (w/v)) als Induktor des L-Carnitinstoffwechsels zugesetzt wurde, überimpft und 2 Tage bei 30°C und 140 upm inkubiert. Die Vorkulturen (0,01 mL) wurde in das Minimalmedium (10 mL), welches zusätzlich 0,02% (w/v) Betain, 0,05% (v/v) Glycerin, 0,50% (w/v) L-Glutamat und entweder 0,75% (w/v) Butyrobetain-methylester (BBMe) oder 0,75% (w/v) Butyrobetain-ethylester (BBEt) enthielt, überimpft und bei 30°C und 140 upm inkubiert. Nach 40 h betrug die OD₆₅₀ von beiden Kulturen ca. 9,0 und die Kulturen befanden sich in der stationären Phase. Nach weiteren 34 h wurden Proben entnommen. Die Zellen wurden zentrifugiert (11 000 upm, 5 min) und der Überstand wurde mittels HPLC auf den Gehalt an Butyrobetain, L-Carnitin und nicht umgesetzten Butyrobetain-ester untersucht. Die Ergebnisse sind in Tabelle 2 zusammengefasst.
Als Kontrollversuch wurden Butyrobetain-methylester (BBMe) und Butyrobetain-ethylester (BBEt) jeweils im gleichen Medium und unter den gleichen Bedingungen, jedoch ohne Zusatz von Zellen, gelagert. Unter diesen Bedingungen war keine Hydrolyse der Butyrobetain-ester zu Butyrobetain nachzuweisen.

**Tabelle 2:**

| **Stamm** | **Edukt** | | **Reaktionsgemisch** | | | | |
|---|---|---|---|---|---|---|---|
| | **BBMe^{a} [mmol/L]** | **BBEt^{a} [mmol/L]** | **BBMe [mmol/L]** | **BBEt [mmol/L]** | **BB^{b} [mmol/L]** | **L-Carnitin [mmol/]** | **Summe^{c} [mmol/L]** |
| HK13 | | 43,1 | - | 26 | 7 | 11 | 44 |
| HK13 | 46,9 | | 28 | - | 15 | <6 | <49 |
| HK1349 | | 43,1 | - | 26 | 6 | 10 | 42 |
| HK1349 | 46,9 | | 27 | - | 13 | 9 | 49 |

| | | | | | | | |
|---|---|---|---|---|---|---|---|
| ^{a} 0,75% (w/v) BBME = 46,9 mmol/L; 0,75% (w/v) BBEt = 43,1 mmol/L. ^{b} BB = Butyrobetain. ^{c} Summe = BBMe oder BBEt + Butyrobetain + L-Carnitin. | | | | | | | |

Die Stämme HK13 (DSM 2903) und HK1349 (DSM 3944) setzten jeweils sowohl Butyrobetain-methylester (BBMe) als auch Butyrobetain-ethylester (BBEt) in 74 h zu ca. 40% um. Bei der Umsetzung des Butyrobetain-methylester ist der Anteil an gebildetem Butyrobetain höher als der an L-Carnitin, während bei der Umsetzung des Butyrobetainethylesters der Anteil an gebildetem L-Carnitin höher ist als der an Butyrobetain.

### Beispiel 3

### Umsetzung von Butyrobetain-methylester (BBMe) und Butyrobetain-ethylester (BBEt) mittels der Stämme HK13 (DSM 2903), HK1349 enthaltend Plasmid pVK100q (DSM 8726) und HK1331b (DSM 3225) zu Butyrobetain und L-Carnitin, Dauer: 144 h

Die Stämme HK13 (DSM 2903), HK1349 enthaltend Plasmid pVK100q (DSM 8726) und HK1331b (DSM 3225) wurden in Analogie zu Beispiel 2 angezogen, jedoch enthielt das Minimalmedium statt 0,75% (w/v) 0,90% (w/v) Butyrobetain-methylester (BBMe) oder Butyrobetain-ethylester (BBEt). Das Vorkulturmedium des Stammes HK1349 enthaltend Plasmid pVK100q (DSM 2903) enthielt zusätzlich Tetracyclin (10 µg/mL). Nach 52 h betrug die OD₆₅₀ von den Kulturen ca. 10,0 und die Kulturen befanden sich in der stationären Phase. Nach weiteren 92 h wurden Proben entnommen. Die Zellen wurden zentrifugiert (11 000 upm, 5 min) und der Überstand wurde mittels HPLC auf den Gehalt an Butyrobetain, L-Carnitin und nicht umgesetzten Butyrobetain-ester untersucht.

Die Stämme HK13 (DSM 2903) und HK1349 enthaltend Plasmid pVK100q (DSM 8726) und HK1331b (DSM 3225) setzten jeweils sowohl Butyrobetain-methylester (BBMe) als auch Butyrobetain-ethylester (BBEt) in 144 h zu ca. 50 % unter Bildung von Butyrobetain und L-Carnitin um.

### Beispiel 4

### Charakterisierung der Hydrolasen im Zellextrakt des Stammes HK1349 (DSM 3944) 4.1 Herstellung Zellextrakt des Stammes HK1349 (DSM 3944)

Ein Inokulum (0,2 mL) des Stammes HK1349 (DSM 3944) wurde in Minimalmedium (25 mL) enthaltend zusätzlich 0,2% (w/v) Betain, 1,0% (w/v) L-Glutamat und 0,2% (w/v) Butyrobetain geimpft und bei 30°C und 140 upm inkubiert bis die OD_{650 nm} 3,0 bis 7,0 betrug. Die so erhaltene Vorkultur (2 mL) wurde in das Minimalmedium (1,5 L) enthaltend zusätzlich 0,2% (w/v) Betain, 1,0% (w/v) L-Glutamat und 0,2% (w/v) Butyrobetain überimpft und bei 30°C und 140 upm inkubiert bis die OD₆₅₀ₙₘ 3,0 bis 7,0 betrug. Die Kultur wurde zentrifugiert (3 500 upm; 30 min; 16°C) und die Zellen wurden in 50 mM Natriumphosphatpuffer (150 mL; pH 7,5) resuspendiert. Die so erhaltene Zellsuspension hatte eine OD_{650 nm} 37.

### 4.2. Untersuchung des Vorliegens der Hydrolasen in der Zelle

Die gemäss Beispiel 4.1. erhaltene Zellsuspension (HK1349 (DSM 3944), OD_{650 nm} 37, 50 mM Natriumphosphat-Puffer, pH 7,5) wurde zentrifugiert (4 000 upm; 10 min; 4°C) und die spezifische Hydrolase-Aktivität des Überstandes wurde mittels Essigsäure-4-nitrophenylester-Assay bestimmt. In einem zweiten Experiment wurde die gemäss Beispiel 4.1. erhaltene Zellsuspension (HK1349 (DSM 3944), OD_{650 nm} 37, 50 mM Natriumphosphat-Puffer, pH 7,5) durch 10 minütige Behandlung mit Ultraschall unter Eisbadkühlung aufgeschlossen, die Zelltrümmer wurden abzentrifugiert (4 000 upm; 10 min; 4°C) und die spezifische Hydrolase-Aktivität des Überstandes wurde mittels Essigsäure-4-nitrophenylester-Assay bestimmt. Die höchste spezifische Hydrolase-Aktivität (0,31 U/mg Protein) wurde als 100%-Wert betrachtet. Die Ergebnisse sind in Tabelle 3 zusammengestellt. Die hydrolytische Aktivität liegt intrazellulär vor.

**Tabelle 3:**

| **Überstand** | **Relative Hydrolase-Aktivität [%]** |
|---|---|
| von ganzen Zellen | 45 |
| nach Zellaufschluss | 100 |

Die gemäss Beispiel 4.1. erhaltene Zellsuspension (HK1349 (DSM 3944), OD_{650 nm} 37, 50 mM Natriumphosphat-Puffer, pH 7,5) wurde durch 10 minütige Behandlung mit Ultraschall unter Eisbadkühlung aufgeschlossen, mit verschiedenen Detergentien (5% (v/v) Detergenz; 30 min; 25°C) inkubiert und die Zelltrümmer wurden abzentrifugiert (4 000 upm; 10 min; 4°C). Die spezifische Hydrolase-Aktivität der Überstände wurde mittels Essigsäure-4-nitrophenylester-Assay bestimmt. Die höchste spezifische Hydrolase-Aktivität (0,30 U/mg Protein) wurde als 100%-Wert betrachtet. Die Ergebnisse sind in Tabelle 4 zusammengestellt. Die höchste spezifische Hydrolase-Aktivität wurde unter Verwendung von Triton X-114 beobachtet.

**Tabelle 4:**

| **Überstand nach Zellaufschluss und Behandlung mit folgendem Detergenz** | **Relative spezifische Hydrolase-Aktivität [%]** |
|---|---|
| - | 70 |
| SDS | 26 |
| Triton X-114 | 100 |
| Triton X-100 | 64 |
| Tween 80 | 80 |
| Lysozym | 64 |

### 4.3. Bestimmung des Molekulargewichtes

Die in Tabelle 3 aufgeführten Überstande sowie die in Tabelle 4 aufgeführten Überstände, welche mit Triton X-114 oder mit Lysozym behandelt wurden, wurden mittels SDS-PAGE untersucht. In allen Überständen wurden eine 22 kDa Hydrolase und eine 33 kDa Hydrolase gefunden.

### 4.4. Bestimmung der Substratspezifität

Die gemäss Beispiel 4.1. erhaltene Zellsuspension (HK1349 (DSM 3944), OD_{650 nm} 37, 50 mM Natriumphosphat-Puffer, pH 7,5) wurde durch 10 minütige Behandlung mit Ultraschall unter Eisbadkühlung aufgeschlossen, mit Triton X-114 (5% (v/v); 30 min; 25°C) inkubiert und dann zentrifugiert (4 000 upm; 10 min; 4°C). Der Überstand wurde lyophilisiert. Der lyophilisierte Überstand (100 mg) wurde in Natriumphosphatpuffer (50 mM; pH 7,5; 1,0 mL) gelöst und zu Emulsionen von Essigsäure-ethylester, Buttersäure-ethylester und Buttersäure-methylester (5% (v/v) Ester emulgiert mittels eines Ultraturrax in destilliertem Wasser enthaltend 2% (w/v) Gummi arabicum; 20 mL) bei 37°C gegeben. Die bei der Hydrolyse des Esters freigesetzte Säure wird mit NaOH (0,01 M) unter Verwendung eines pH-Stats titriert. 1 U (Unit) entspricht der Menge an Säure (in µmol), die pro Minute freigesetzt wird. Die höchste spezifische Hydrolase-Aktivität (0,039 U/mg Protein) wurde als 100%-Wert betrachtet. Die Ergebnisse sind in Tabelle 5 zusammengestellt.

**Tabelle 5:**

| **Substrat** | **Spezifische Hydrolase-Aktivität [U/mg Protein]** | **Relative spezifische Hydrolase-Aktivität [%]** |
|---|---|---|
| Essigsäure-Ethylester | 0,039 | 100 |
| Buttersäure-Ethylester | 0,028 | 72 |
| Buttersäure-Methylester | 0,038 | 97 |

### 4.5. Bestimmung des pH-Optimums

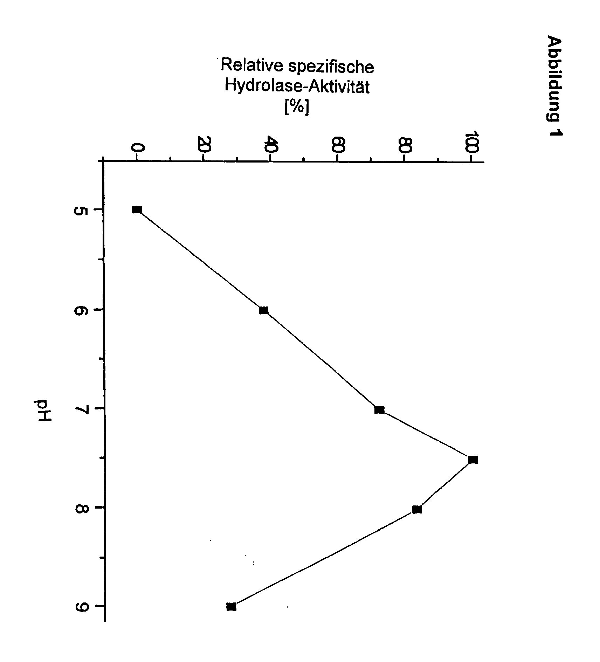

Der gemäss Beispiel 4.4. erhaltene lyophilisierte Überstand (100 mg) wurde in Natriumphosphat-Puffer (50 mM; pH 7,5; 1,0 mL) gelöst. Diese Lösung (50 µL) wurde mit Natriumphosphat-Puffern unterschiedlicher pH-Werte (50 mM; pH 5,0 bis 9,0; 850 µL) verdünnt und mit einer Lösung von Essigsäue-4-nitrophenylester (10 mM in Dimethylsulfoxid, 100 µL) bei 25°C versetzt. Die Absorption des während der Hydrolyse gebildeten 4-Nitrophenols (ε = 15 × 10³ × M⁻¹ cm⁻¹) wurde bei 410 nm gemessen. Die Ergebnisse sind in Abbildung 1 dargestellt. Das pH-Optimum beträgt 7,5.

### 4.6. Bestimmung der Temperaturstabilität

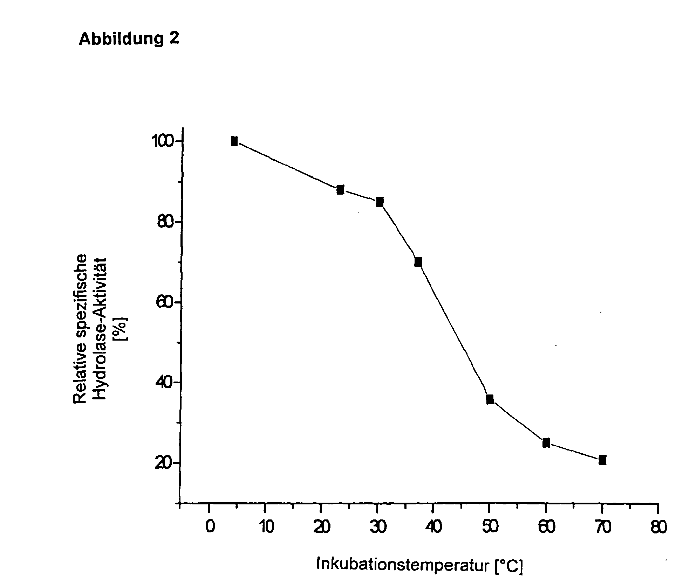

Die gemäss Beispiel 4.1. erhaltene Zellsuspension (HK1349 (DSM 3944), OD_{650 nm} 37, 50 mM Natriumphosphat-Puffer, pH 7,5) wurde durch 10 minütige Behandlung mit Ultraschall unter Eisbadkühlung aufgeschlossen, mit Triton X-114 (5% (v/v); 30 min; 25°C) inkubiert und dann zentrifugiert (4 000 upm; 10 min; 4°C). Der Überstand wurde bei Temperaturen im Bereich von 5 bis 70°C 16 h inkubiert. Nach der Inkubation wurde die spezifische Hydrolase-Aktivität mittels Essigsäure-4-nitrophenylester-Assay bestimmt. Die Ergebnisse sind in Abbildung 2 dargestellt. Die spezifische Hydrolase-Aktivität nimmt mit steigender Temperatur ab. Nach 16 stündiger Inkubation bei 30°C liegt noch ca. 85% der ursprünglichen Aktivität vor, nach 16 stündiger Inkubation bei 45°C liegt noch ca. 50% der ursprünglichen Aktivität vor nach 16 stündiger Inkubation bei 50°C liegt nur noch ca. 35% der ursprünglichen Aktivität vor.

### Beispiel 5

### Reinigung der Hydrolasen des Stammes HK1349 (DSM 3944)

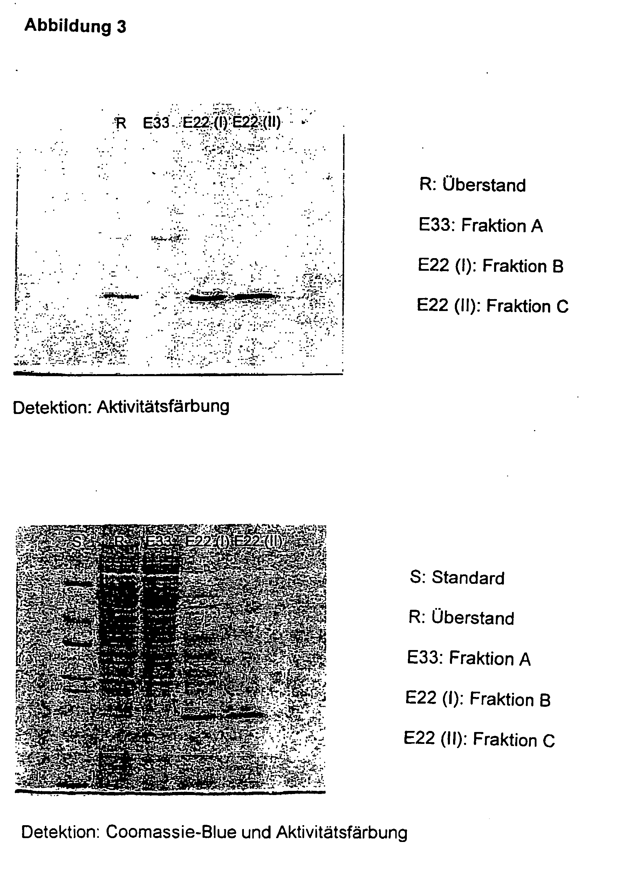

Die gemäss Beispiel 4.1. erhaltene Zellsuspension (HK1349 (DSM 3944), OD_{650 nm} 37, 50 mM Natriumphosphat-Puffer, pH 7,5) wurde durch 10 minütige Behandlung mit Ultraschall unter Eisbadkühlung aufgeschlossen, mit Triton X-114 (5% (v/v); 30 min; 25°C) inkubiert und dann zentrifugiert (4 000 upm; 10 min; 4°C). Proben des Überstandes (10 mL) wurden mittels Gel-Filtration (Sephacryl^{™} S-200, 100 cm³; Elutionsmittel: Natriumphosphatpuffer (50 mM; pH 7,5); Durchflussrate: 1 mL/min) gereinigt.
Von den einzelnen Fraktionen (2 mL) wurde die spezifische Hydrolase-Aktivität mittels Essigsäure-4-nitrophenylester-Assay bestimmt. Die Fraktionen, welche eine Hydrolase-Aktivität besaßen, wurden mittels SDS-PAGE analysiert. Die Fraktionen, welche nur eine Hydrolase mit dem Molekulargewicht von 33 kDa enthielten, wurden zusammengegeben (Fraktion A). Die Fraktionen welche nur eine Hydrolase mit dem Molekulargewicht von 22 kDa enthielten, und die laut SDS-PAGE "verunreinigter" waren, wurden zusammengegeben (Fraktion B), sowie die Fraktionen welche nur eine Hydrolase mit dem Molekulargewicht von 22 kDa enthielten, und die laut SDS-PAGE "reiner" waren, wurden zusammengegeben (Fraktion C). Fraktionen, die "reiner" waren, beinhalteten einen geringeren Anteil an Fremdproteinen als die Fraktionen die "verunreinigter" waren. Von den Fraktionen A, B und C wurde die spezifische Hydrolase-Aktivität erneut mittels Essigsäure-4-nitrophenylester-Assay bestimmt und diese Proteinlösungen wurden erneut mittels SDS-PAGE analysiert. Die Ergebnisse sind in Tabelle 6 und Abbildung 3 zusammengestellt.

**Tabelle 6:**

| **Proteinlösung** | **Spezifische Hydrolase-Aktivität [U/mg Protein]** |
|---|---|
| Überstand | 0,080 |
| Fraktion A | 0,122 |
| Fraktion B | 0,260 |
| Fraktion C | 0,199 |

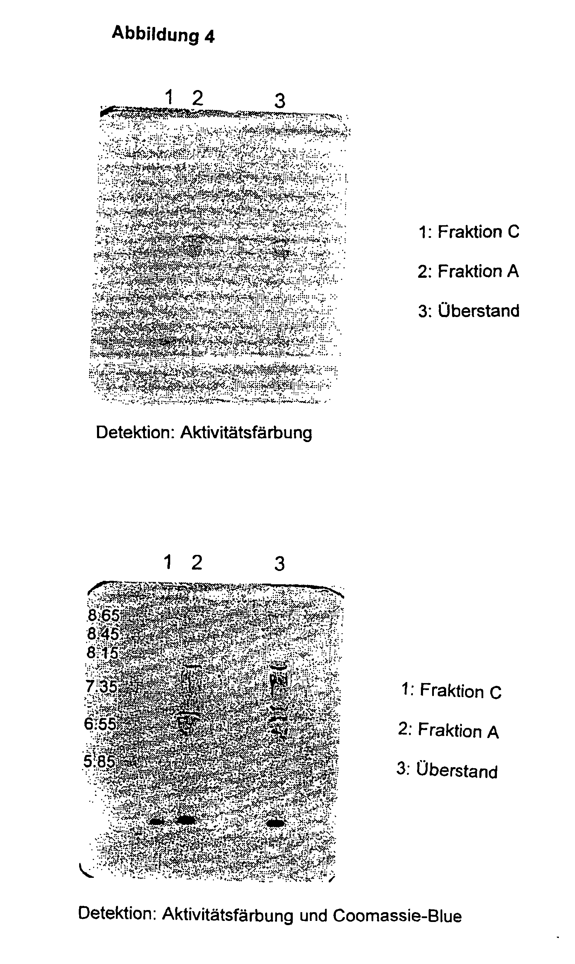

Die Fraktion A enthaltend die 33 kDa Hydrolase und die Fraktion C enthaltend die 22 kDa Hydrolase wurden mittels Ultrafiltration aufkonzentriert. Die isoelektrischen Punkte der 22°kDa Hydrolase und der 33 kDa Hydrolase wurden mit dem PhastSystem (Amersham Pharmacia) nach Anleitung des Herstellers bestimmt. Hierzu wurden die Fraktionen A und C mit einem Gel, welches einen pH-Gradienten von 3 bis 9 aufweist, analysiert. Die Ergebnisse sind in Abbildung 4 dargestellt. Fraktion C wurde zusätzlich mit einem Gel, welches einen pH-Gradienten von 4 bis 6,5 aufweisst, analysiert. Die Detektion erfolgte analog der Detektion bei der SDS-PPAGE. Die 33 kDa Hydrolase hat einen pI von 6,58, die 22 kDa Hydrolase hat einen pI von 4,15.

### Beispiel 6

### Umsetzung von Butyrobetain-methylester (BBMe) zu Butyrobetain mittels Fraktion A und Fraktion C

Fraktion C aus Beispiel 5 (1,0 mL) wurde mittels Centricons (2 mL, 10 kDa; 5 000 upm; 60 min; 4°C) auf ein 1/10 des ursprünglichen Volumens aufkonzentriert. Eine Reaktionslösung bestehend aus Butyrobetain-methylester (BBMe) (50 µmol) und Natriumphosphat-Puffer (50 mM; pH 7,5; 500 µL) wurde mit der aufkonzentrierten Fraktion C (100 µL) bei 37°C versetzt. Die Umsetzung von Butyrobetain-methylester zu Butyrobetain wurde qualitativ mittels Dünnschichtchromatographie verfolgt.
Die 22 kDa Hydrolase der Fraktion C setzte Butyrobetain-Methylester (BBMe) in 1 h nachweisbar zu Butyrobetain um und in 60 h zu ca. 15% (geschätzt).
In Analogie wurden Fraktionen A aus Beispiel 5 aufkonzentriert und mit Butyrobetain-methylester (BBMe) umgesetzt. Die 33 kDa Hydrolase der Fraktion A setzte Butyrobetain-methylester (BBMe) nicht zu Butyrobetain um.

### Beispiel 7

### Hydrolyse von Butyrobetain-methylester (BBMe) zu Butyrobetain mittels den Esterasen PFE I, PFE II und SDE

In Analogie zu Beipiel 6 wurden Butyrobetain-methylester (BBMe) mit PFE I aus *Pseudomonas fluorescens* (Bornscheuer et al. *J. Biotechnol.* **1998**, *60,* 105-111), Esterase PFE II aus *Pseudomonas fluorescens* (DSM 50106, Khalameyer et al *Appl. Environm. Microbiol.* **1999,** *65,* 477-482) und mit Esterase SDE aus *Streptomyces diastatochromogenes* (erhältlich z. B. von Jülich Fine Chemicals, Bomscheuer et al. *Biotechnol. Letters* **1999**, *21,* 101-104, Tesch et al. *J. Bacteriol.* **1996**, *178*, 1858-1865) (jeweils ca. 6 mg) umgesetzt.

Alle drei Esterasen hydrolysierten den Butyrobetain-methylester (BBMe), wobei die Esterase PFE II die höchste Aktivität aufweist. Die Esterasen PFE II und SDE setzten Butyrobetain-methylester (BBMe) bereits nach 1 h nachweisbar zu Butyrobetain um.

### Beispiel 8

### Hydrolyse von Butyrobetain-methylester (BBMe) zu Butyrobetain mittels käuflicher Hydrolasen

Eine Reaktionslösung bestehend aus Butyrobetain-methylester (BBMe) (1,0% (w/v)) in Kaliumphosphatpuffer (10 mM; pH 7,0; 2,0 mL) wurde mit unterschiedlichen Hydrolasen (10 bis 15 mg oder 20 µL) bei 25°C versetzt. Der pH-Wert des Reaktionsgemisches wird mittels einer pH-Sonde 24 h verfolgt. Durch Hydrolyse des gebildetes Butyrobetain sollte der pH-Wert sinken. Bei den Reaktionen, wo ein Abfall des pH-Wertes beobachtet werden konnte, wurde der Gehalt an Butyrobetain-methylester (BBMe) und Butyrobetain im Reaktionsgemisch mittels HPLC bestimmt. Die Ergebnisse sind in Tabelle 7 zusammengestellt. Die Lipase aus *Bacillus thermocatenulatus* (Roche diagnostics) die Schweineleber Esterasen (von Fluka und Roche diagnostics) setzten am meisten Butyrobetain-methylester (BBMe) zu Butyrobetain um. Die Lipase aus *Bacillus thermoeatenulatus* wurde bereits kloniert (Schmid-Dannert et al., *Biochim. Biophys. Acta* **1996**, *1301,* 105-114).

**Tabelle 8:**

| **Hydrolase** | **BBMe [µmol/mL]** | **Butyrobetain [µmol/mL]** |
|---|---|---|
| - | 78,1 | 1,6 |
| Schweineleber Esterase (Fluka) | 68,9 | 5,9 |
| Lipase aus *Bacillus thermocatenulatus* (Roche diagnostics) | 71,1 | 6,3 |
| Schweineleber Esterase (Roche diagnostics) | 71,2 | 5,9 |
| Subtilisin A60 (Novo Nordisk) | 73,8 | 2,5 |
| Chirazym L2 (Roche diagnostics) | 171,8 | 4,6 |

## Patentansprüche

1. Hydrolase, die 4-Butyrobetain-methylester als Substrat zu 4-Butyrobetain umsetzt, **gekennzeichnet durch**:
a) einen pI von 4,15 ± 0,30 und
b) ein Molekulargewicht von 22,0 ± 2,0 kDa, bestimmt mittels SDS-PAGE.

2. Verfahren zur Herstellung von L-Carnitin, worin ein Betain-ester der allgemeinen Formel 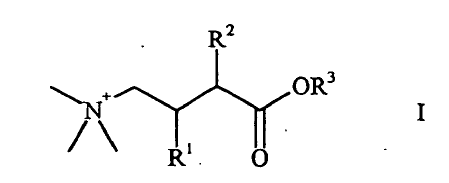 worin R¹ und R² entweder Wasserstoff oder zusammen eine Bindung bedeuten und R³ gegebenenfalls substituiertes C₁₋₁₀-Alkyl, gegebenenfalls substituiertes Aryl oder gegebenenfalls substituiertes Arylalkyl bedeutet, mittels einer Hydrolase, die einen Betain-ester der allgemeinen Formel I als Substrat zum Betain umsetzt, oder mittels eines eine solche Hydrolase enthaltenden Mikroorganismus zum entsprechenden Betain der allgemeinen Formel 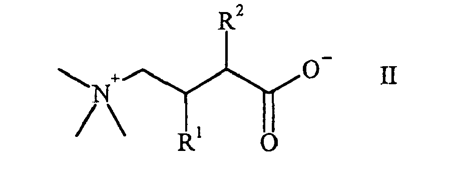 worin R¹ und R² die oben genannte Bedeutung haben, umgesetzt wird, und dieses Betain mittels eines Mikroorganismus, der befähigt ist, das Betain der Formel II zu L-Carnitin umzusetzen, zu L-Carnitin umgesetzt wird.

3. Verfahren nach Anspruch 2 worin die Hydrolase eine Hydrolase nach Anspruch 1 ist.

4. Verfahren nach Anspruch 2 oder 3, worin der die Hydrolase enthaltende Mikroorganismus gleichzeitig der Mikroorganismus ist, mit dem das gebildete Betain der allgemeinen Formel II zu L-Carnitin umgesetzt wird.

5. Verfahren nach einem der Ansprüche 2 bis 4, worin der die Hydrolase enthaltende Mikroorganismus ein natürlich vorkommender oder ein rekombinanter Mikroorganismus ist.

6. Verfahren zur Herstellung von Betainen der allgemeinen Formel 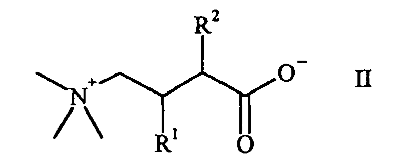 worin R¹ und R² die in Anspruch 5 genannte Bedeutung haben, worin ein Betain-ester der allgemeinen Formel 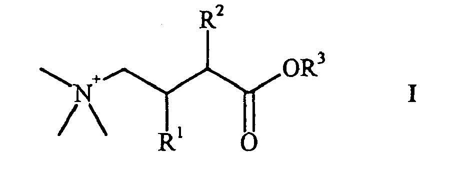 worin R¹ und R² die oben genannte Bedeutung haben und R³ die in Anspruch 5 genannte Bedeutung hat, mittels einer Hydrolase, die einen Betain-ester der allgemeinen Formel I hydrolysiert, oder mittels eines eine solche Hydrolase enthaltenden Mikroorganismus zum entsprechenden Betain der allgemeinen Formel II umgesetzt wird.

7. Verfahren nach einem der Ansprüche 2 bis 6 worin der Betain-ester der allgemeinen Formel I derart zugeführt wird, dass die Konzentration des Betain-esters im Reaktionsgemisch unter 10% (w/v) bleibt.

8. Verfahren nach einem der Ansprüche 2 bis 7 worin der pH-Wert im Bereich von 6 bis 8 und die Temperatur im Bereich von 15 bis 50°C liegt.

9. Verfahren nach einem der Ansprüche 2 bis 8 worin ein Betain-ester der allgemeinen Formel I 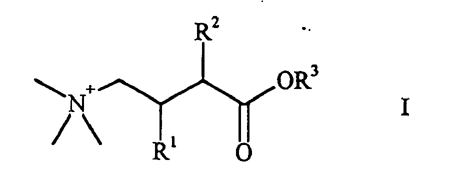 eingesetzt wird, worin R¹ und R² entweder Wasserstoff oder zusammen eine Bindung bedeuten und R³ Methyl bedeutet.

10. Verwendung einer Hydrolase, die 4-Butyrobetain-methylester zu 4-Butyrobetain umsetzt, in einem Verfahren zur Herstellung von L-Carnitin

11. Verwendung von Mikroorganismen, welche eine Hydrolase enthalten, die 4-Butyrobetain-methylester zu 4-Butyrobetain umsetzt, zur Herstellung von L-Carnitin.

## Claims

1. Hydrolase which converts 4-butyrobetaine methyl ester as substrate to 4-butyrobetaine, **characterized by**:
a) a pI of 4.15 ± 0.30 and
b) a molecular weight of 22.0 ± 2.0 kDa, determined by SDS-PAGE.

2. Method for producing L-carnitine in which a betaine ester of the general formula 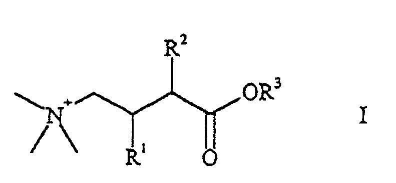 where R¹ and R² are either hydrogen or together are a bond and R³ is unsubstituted or substituted C₁₋₁₀alkyl, unsubstituted or substituted aryl or unsubstituted or substituted arylalkyl, is converted using a hydrolase which converts a betaine ester of the general formula I as substrate to betaine, or using a microorganism containing such a hydrolase, to the corresponding betaine of the general formula 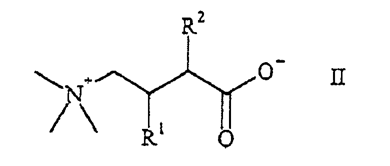 where R¹ and R² have the meaning specified above, and this betaine is converted to L-carnitine using a microorganism which is able to convert the betaine of the formula II to L-carnitine.

3. Method according to Claim 2 in which the hydrolase is a hydrolase according to Claim 1.

4. Method according to Claim 2 or 3 in which the hydrolase-containing microorganism is simultaneously the microorganism which converts the betaine formed of the general formula II to L-carnitine.

5. Method according to one of Claims 2 to 4 in which the hydrolase-containing microorganism is a naturally occurring or recombinant microorganism.

6. Method for producing betaines of the general formula 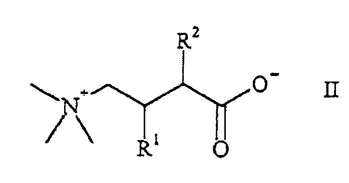 where R¹ and R² have the meaning specified in Claim 5, in which a betaine ester of the general formula 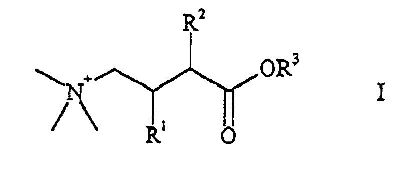 where R¹ and R² have the meaning specified above and R³ has the meaning specified in Claim 5, is converted to the corresponding betaine of the general formula II using a hydrolase which hydrolyses a betaine ester of the general formula I or using a microorganism containing such a hydrolase.

7. Method according to one of Claims 2 to 6 in which the betaine ester of the general formula I is fed in such a manner that the concentration of the betaine ester in the reaction mixture remains below 10% (w/v).

8. Method according to one of Claims 2 to 7 in which the pH is in the range from 6 to 8 and the temperature is in the range from 15 to 50°C.

9. Method according to one of Claims 2 to 8 in which a betaine ester of the general formula I 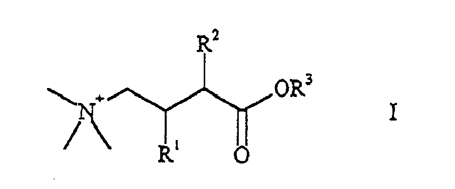 is used, where R¹ and R² either are hydrogen or together are a bond and R³ is methyl.

10. Use of a hydrolase which converts 4-butyrobetaine methyl ester to 4-butyrobetaine in a method for producing L-carnitine.

11. Use of microorganisms containing a hydrolase which converts 4-butyrobetaine methyl ester to 4-butyrobetaine for producing L-carnitine.

## Revendications

1. Hydrolase qui convertit l'ester méthylique de la 4-butyrobétaïne, servant de substrat, en 4-butyrobétaïne, **caractérisée par**
a) un pI de 4,15 ± 0,30, et
b) une masse moléculaire de 22,0 ± 2,0 kDa, déterminée par SDS-PAGE.

2. Procédé de préparation de L-carnitine, dans lequel on fait réagir un ester de bétaïne de formule générale 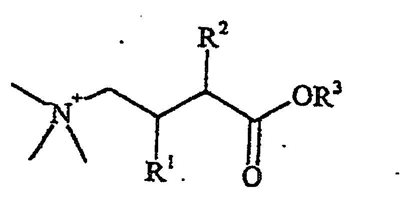 dans laquelle R¹ et R² sont des atomes d'hydrogène, ou forment ensemble une liaison, et R³ représente un radical alkyle en C₁₋₁₀ éventuellement substitué, aryle éventuellement substitué ou arylalkyle éventuellement substitué, à l'aide d'une hydrolase qui convertit en bétaïne un ester de bétaïne de formule générale I servant de substrat, ou à l'aide d'un microorganisme contenant une telle hydrolase, pour obtenir la bétaïne correspondante de formule générale 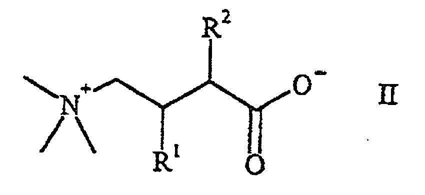 dans laquelle R¹ et R² ont les significations données ci-dessus, et on convertit en L-carnitine cette bétaïne au moyen d'un microorganisme à même de convertir la bétaïne de formule II en L-carnitine.

3. Procédé selon la revendication 2, dans lequel l'hydrolase est une hydrolase selon la revendication 1.

4. Procédé selon la revendication 2 ou 3, dans lequel le microorganisme contenant l'hydrolase est simultanément le microorganisme à l'aide duquel la bétaïne formée de formule générale II est convertie en L-carnitine.

5. Procédé selon l'une des revendications 2 à 4, dans lequel le microorganisme contenant l'hydrolase est un microorganisme naturel ou un microorganisme recombinant.

6. Procédé de préparation de bétaïnes de formule générale 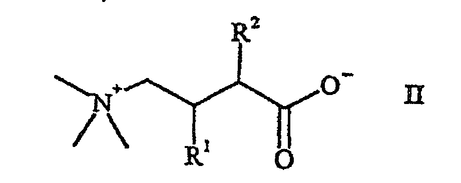 dans laquelle R¹ et R² ont les significations données dans la revendication 5, procédé dans lequel on fait réagir, pour obtenir la bétaïne correspondante de formule II, un ester de bétaïne de formule générale 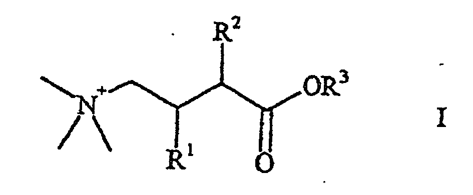 dans laquelle R¹ et R² ont les significations données ci-dessus et R³ a les significations données dans la revendication 5, à l'aide d'une hydrolase qui hydrolyse un ester de bétaïne de formule générale I, ou à l'aide d'un microorganisme contenant une telle hydrolase.

7. Procédé selon l'une des revendications 2 à 6, dans lequel l'ester de bétaïne de formule générale I est amené de telle sorte que la concentration de l'ester de bétaïne dans le mélange réactionnel soit inférieure à 10 % (m/v).

8. Procédé selon l'une des revendications 2 à 7, dans lequel le pH est compris dans la plage de 6 à 8, et la température est comprise dans la plage de 15 à 50°C.

9. Procédé selon l'une des revendications 2 à 8, dans lequel on utilise un ester de bétaïne de formule générale I 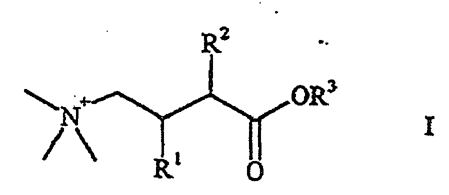 dans laquelle R¹ et R² sont des atomes d'hydrogène, ou représentent ensemble une liaison, et R³ est le groupe méthyle.

10. Utilisation d'une hydrolase qui convertit l'ester méthylique de la 4-butyrobétaïne en 4-butyrobétaïne dans un procédé de préparation de L-carnitine.

11. Utilisation de microorganismes contenant une hydrolase qui convertit l'ester méthylique de la 4-butyrobétaïne en 4-butyrobétaïne, pour préparer la L-carnitine
